# EUROPEAN PATENT APPLICATION

(11) **EP 4 501 292 A1**
(43) Date of publication of application: **05.02.2025**
(21) Application number: 23826990.6
(22) Date of filing: 07.06.2023
(51) Int. Cl.: A61F 13/15, A61F 13/49, A61F 13/496, A61F 13/51

(54) **PANTS-TYPE ABSORBENT ARTICLE**

(30) Priority: 20.06.2022 JP 2022099151
(71) Applicant: Unicharm Corporation, Ehime 799-0111 (JP)
(72) Inventor: TANAKA, Suguru, Kanonji-shi, Kagawa 769-1602 (JP); SHIMAZU, Takeshi, Kanonji-shi, Kagawa 769-1602 (JP); ISHII, Yudai, Kanonji-shi, Kagawa 769-1602 (JP)
(74) Representative: Dolleymores
(86) International application number: PCT/JP2023/021199
(87) International publication number: WO 2023/248803

(57) **Abstract**

This pants-type absorbent article (1) has an absorbent body (10) and a waistline member (20) that is flexible in the right and left directions. The waistline member (20) comprises a front-side waistline part (FA) and a back-side waistline part (BA). The front-side waistline part (FA) and the back-side waistline part (BA) are joined to each other by a pair of side joints (40) that are provided on either side in the right and left directions. The difference between the magnitude of force obtained when having stretched the waistline member (20) from a natural state until the length thereof becomes equal to 80% of the maximally stretched length thereof and the magnitude of force obtained when having retracted the waistline member (20) from the maximally stretched state until the length thereof becomes equal to 80% of the maximally stretched length is 4.0 N or less.

## Description

### FIELD

The present invention relates to an underpants-shaped absorbent article.

### BACKGROUND

Conventionally, there are known underpants-shaped absorbent articles in which a waist member has a plurality of elastic members that have the stretchability in the lateral direction. For example, Patent Document 1 discloses an underpants-shaped diaper in which a hot-melt adhesive is applied to the outer surfaces of elastic stretch members 12C, which are sandwiched between an outer layer 12S and an inner layer 12H that constitute an exterior sheet 12 (corresponding to the waist member), and the two layers 12S and 12H are bonded together with the adhesive and the elastic stretch members 12C.

### [CITATION LIST]

### [PATENT LITERATURE]

[Patent Document 1] Japanese Patent Application Publication No. 2012-61348

### SUMMARY

### [TECHNICAL PROBLEM]

In such a underpants-shaped absorbent article, when being worn, the length of the waist member in the lateral direction (i.e. the circumference of the waist opening) is not always kept constant, and the waist member may stretch/contract in the lateral direction depending on the body movements and breathing of the wearer. In particular, when the waist member is worn with the highly-stretched state, where the length of the waist member in the lateral direction is approximately 80% of its most stretched state, the constriction force became larger around the wearer's torso as accompanied with the stretching/contracting of the waist member, making it difficult to wear comfortably.

The present invention was achieved in light of conventional problems such as that described above, an aspect of the present invention is to provide an underpants-shaped absorbent article that can be worn comfortably even when the stretch ratio of the waist member is high.

### [SOLUTION TO PROBLEM]

A main aspect of the present invention of achieving the above-described aspect is an underpants-shaped absorbent article having a vertical direction, a lateral direction, and a front-back direction that intersect with each other, the underpants-shaped absorbent article including: a liquid-absorbent absorbent main body; and a waist member that has stretchability in the lateral direction, the waist member having a front waist portion provided on a front side in the front-back direction and a back waist portion provided on a back side in the front-back direction, the front waist portion and the back waist portion being joined to each other by a pair of side joining portions that are provided on two sides in the lateral direction, in the lateral direction, concerning a magnitude of force when the waist member is stretched from a natural state until a length of the waist member reaches to 80% of a most stretched length, and concerning a magnitude of force when the waist member is contracted from the most stretched state until the length of the waist member reaches 80% of the most stretched length, a difference between the magnitude of force when being stretched and the magnitude of force when being contracted being 4.0 N or less.

Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article that can be worn comfortably even when the stretch ratio of the waist member is high.

### BRIEF DESCRIPTION OF DRAWINGS

FIG. 1 is a schematic perspective view showing an example of the configuration of a diaper 1.
FIG. 2 is a schematic plan view of the diaper 1 in a stretched state as viewed from a skin side in a thickness direction.
FIG. 3 is a schematic cross-sectional view taken along line A-A of FIG. 2.
FIG. 4 is a plan view and a cross-sectional view of an absorbent main body 10.
FIG. 5 is a diagram illustrating the range in a waist member 20 within which a plurality of welded portions 50 are formed.
FIG. 6 is a diagram illustrating an example of the arrangement of the welded portions 50.
FIGS. 7A and 7B are diagrams illustrating the principle by which waist elastic members 25 are attached to the waist member 20 by the welded portions 50.
FIG. 8 is a diagram illustrating the concept of a tensile test for measuring the magnitude of the constriction force of the waist member 20.
FIG. 9 is a table showing the results of the tensile test on the diaper 1 and Comparative Examples A to F.
FIG. 10 is a graph showing the relationship between the stretch ratio of the waist member and the magnitude of force during stretching/contracting for the diaper 1 and Comparative Examples A and B.
FIG. 11 is a table showing the results of the tensile test on the lower portions FA2 of the diaper 1 and of Comparative Examples A to F.
FIG. 12 is a graph showing the relationship between the stretch ratio in the lower portion FA2 of the waist member and the magnitude of force during stretching/contracting for the diaper 1 and Comparative Examples A and B.
FIG. 13 is a table showing the results of the tensile test on the upper portions FA1 of the diaper 1 and of Comparative Examples A to F.
FIG. 14 is a graph showing the relationship between the stretch ratio in the upper portion FA1 of the waist member and the magnitude of force during stretching/contracting for the diaper 1 and Comparative Examples A and B.

### DESCRIPTION OF EMBODIMENTS

At least the following matters will be clear with the description of this specification and the attached drawings.

### (Aspect 1)

An underpants-shaped absorbent article having a vertical direction, a lateral direction, and a front-back direction that intersect with each other, the underpants-shaped absorbent article including: a liquid-absorbent absorbent main body; and a waist member that has stretchability in the lateral direction, the waist member having a front waist portion provided on a front side in the front-back direction and a back waist portion provided on a back side in the front-back direction, the front waist portion and the back waist portion being joined to each other by a pair of side joining portions that are provided on two sides in the lateral direction, concerning a magnitude of force when the waist member is stretched in the lateral direction from a natural state until a length of the waist member reaches to 80% of a most stretched length, and concerning a magnitude of force when the waist member is contracted in the lateral direction from the most stretched state until the length of the waist member reaches 80% of the most stretched length, a difference between the magnitude of force when being stretched and the magnitude of force when being contracted being 4.0 N or less.

According to the underpants-shaped absorbent article of aspect 1, when the waist member is worn in the highly-stretched state (the stretch ratio 80%), when the waist member stretches or contracts in the lateral direction, the difference in the magnitude of force between when being stretched and when being contracted is 4.0 N or less, and therefore the constriction force acting around the wearer's waist is smaller than when the difference in the magnitude of force is greater than 4.0 N. Therefore, even in the highly-stretched state, it is possible to wear it comfortably.

### (Aspect 2)

The underpants-shaped absorbent article according to aspect 1, wherein, concerning a magnitude of force when the waist member is stretched in the lateral direction from the natural state until the length of the waist member reaches to 60% of the most stretched length, and concerning a magnitude of force when the waist member is contracted in the lateral direction from the most stretched state until the length of the waist member reaches 60% of the most stretched length, a difference between the magnitude of force when being stretched and the magnitude of force when being contracted is 2.5 N or less.

According to the underpants-shaped absorbent article of aspect 2, when the waist member is worn in the low-stretched state (the stretch ratio 60%), the difference in the magnitude of force between when being stretched and when being contracted is 2.5 N or less, the constriction force is smaller than when the difference in the magnitude of force is greater than 2.5 N. Therefore, even in the low-stretched state, it is possible to wear it comfortably.

### (Aspect 3)

The underpants-shaped absorbent article according to aspect 1 or 2, wherein, concerning a magnitude of force when the waist member is stretched in the lateral direction from the natural state until the length of the waist member reaches to 85% of the most stretched length, and concerning a magnitude of force when the waist member is contracted in the lateral direction from the most stretched state until the length of the waist member reaches 85% of the most stretched length, a difference between the magnitude of force when being stretched and the magnitude of force when being contracted is 4.0 N or less.

According to the underpants-shaped absorbent article of aspect 3, when the waist member is worn in the highly-stretched state (the stretch ratio 85%), the difference in the magnitude of force between when being stretched and when being contracted is 4.0 N or less, the constriction force is smaller than when the difference in the magnitude of force is greater than 4.0 N. Therefore, even in the maximum stretched state, which is expected in normal use, it is possible to wear it comfortably.

### (Aspect 4)

The underpants-shaped absorbent article according to any one of aspects 1 to 3, wherein, concerning a magnitude of force when the waist member is contracted in the lateral direction from the most stretched state until the length of the waist member reaches 50% of the most stretched length, and concerning a magnitude of force when the waist member is stretched in the lateral direction from the natural state until the length of the waist member reaches to 50% of the most stretched length, a ratio obtained by dividing the magnitude of force when being contracted by the magnitude of force when being stretched is 50% or more.

According to the underpants-shaped absorbent article of aspect 4, the magnitude of force acting when the waist member contracts is 50% or more of the magnitude of force acting when the waist member 20 stretches. The force during contraction is less likely to become small compared to the case of being less than 50%. Therefore, when being worn in the low-stretched state (50%), it becomes easier to prevent a shortage of the constriction force, which causes the waist member to shift out of place.

### (Aspect 5)

The underpants-shaped absorbent article according to any one of aspects 1 to 4, wherein, concerning a magnitude of force when the waist member is contracted in the lateral direction from the most stretched state until the length of the waist member reaches 60% of the most stretched length, and concerning a magnitude of force when the waist member is stretched in the lateral direction from the natural state until the length of the waist member reaches to 60% of the most stretched length, a ratio obtained by dividing the magnitude of force when being contracted by the magnitude of force when being stretched is 60% or more.

According to the underpants-shaped absorbent article of aspect 5, the magnitude of force acting when the waist member contracts is 60% or more of the magnitude of force acting when the waist member 20 stretches. The force during contraction is less likely to become small compared to the case of being less than 60%. Therefore, when being worn in the low-stretched state (60%), it becomes easier to prevent a shortage of the constriction force, which causes the waist member to shift out of place.

### (Aspect 6)

The underpants-shaped absorbent article according to any one of aspects 1 to 5, wherein a rate of increase in a magnitude of force when the waist member is stretched in the lateral direction from 50% of the most stretched length to 85% of the most stretched length is 30 N or less.

According to the underpants-shaped absorbent article of aspect 6, the rate of increase in force when the waist member is stretched in the range of 50% to 85% of the stretch ratio is 30 N or less, making the waist member more likely to stretch gradually compared to the case where the rate of increase in force is greater than 30 N. This prevents a sudden increase in the constriction force of the waist member, and makes it easier to wear the underpants-shaped absorbent article comfortably.

### (Aspect 7)

The underpants-shaped absorbent article according to aspect 6, wherein a rate of increase in a magnitude of force when the waist member is stretched in the lateral direction from 75% of the most stretched length to 85% of the most stretched length is 44 N or less.

According to the underpants-shaped absorbent article of aspect 7, the rate of increase in force when the waist member is stretched in the range of 75% to 85% of the stretch ratio is 44 N or less, making the waist member more likely to stretch gradually compared to the case where the rate of increase in force is greater than 44 N. Therefore, even in the highly-stretched state, it is possible to wear it comfortably.

### (Aspect 8)

The underpants-shaped absorbent article according to any one of aspects 1 to 7, wherein concerning the magnitude of force when the waist member is stretched in the lateral direction from the natural state until the length of the waist member reaches to 80% of the most stretched length, and concerning the magnitude of force when the waist member is contracted in the lateral direction from the most stretched state until the length of the waist member reaches 80% of the most stretched length, a difference between the magnitude of force when being stretched and the magnitude of force when being contracted, in a region located below a center position of the pair of side joining portions in the vertical direction, is 1.5 N or less.

According to the underpants-shaped absorbent article in aspect 8, in the lower portion of the waist member, which overlaps with the absorbent main body and the absorbent core, the difference in the magnitude of force between stretching and contraction is 1.5 N or less. The difference in magnitude of force between stretching and contraction is smaller compared to the case of being greater than 1.50 N. Therefore, it is possible to wear it comfortably even in the highly-stretched state, and it becomes easier to prevent the absorbent main body and the absorbent core from shifting out of place.

### (Aspect 9)

The underpants-shaped absorbent article according to any one of aspects 1 to 8, wherein concerning the magnitude of force when the waist member is stretched in the lateral direction from the natural state until the length of the waist member reaches 80% of the most stretched length, and concerning the magnitude of force when the waist member is contracted in the lateral direction from the most stretched state until the length of the waist member reaches 80% of the most stretched length, the difference between the magnitude of force when being stretched and the magnitude of force when being contracted, in the region located below the center position of the pair of side joining portions in the vertical direction is smaller than a difference between the magnitude of force when being stretched and the magnitude of force when being contracted, in a region located above the center position of the pair of side joining portions in the vertical direction.

According to the underpants-shaped absorbent article of aspect 9, when the waist member is worn in the highly-stretched state (80%), the difference in force between stretching and contracting in the lower portion of the waist member is smaller than the difference in force between stretching and contracting in the upper portion of the waist member. This allows the comfortable feeling of the waist member to be maintained. Simultaneously, in the lower portion, which overlaps with the absorbent main body and the like, a large stretching force in the lateral direction is prevented from acting on the absorbent main body, making the absorbent main body less likely to shift out of position.

### (Aspect 10)

The underpants-shaped absorbent article according to any one of aspects 1 to 9, wherein in a region located above a center position of the pair of side joining portions in the vertical direction, concerning a magnitude of force when the waist member is stretched in the lateral direction from the natural state until the length of the waist member reaches to 85% of the most stretched length, and concerning a magnitude of force when the waist member is contracted in the lateral direction from the most stretched state until the length of the waist member reaches 85% of the most stretched length, a difference between the magnitude of force when being stretched and the magnitude of force when being contracted is 1.35 N or less.

According to the underpants-shaped absorbent article of aspect 10, even when worn in the maximum stretched state (85%) expected in normal use, the difference in magnitude of force between stretching and contracting is 1.35 N or less in the upper portion of the waist member, where the wearer is likely to feel constriction. compared to the case where the difference in magnitude of force is greater than 1.35 N, the constriction force is weaker, making it easier to wear the underpants-shaped absorbent article comfortably.

### (Aspect 11)

The underpants-shaped absorbent article according to any one of aspect 1 to 10, wherein in a region located below a center position of the pair of side joining portions in the vertical direction, concerning a magnitude of force when the waist member is contracted in the lateral direction from the most stretched state until the length of the waist member reaches 50% of the most stretched length, and concerning a magnitude of force when the waist member is stretched in the lateral direction from the natural state until the length of the waist member reaches to 50% of the most stretched length, a ratio obtained by dividing the magnitude of force when being contracted by the magnitude of force when being stretched is 50% or more.

According to the underpants-shaped absorbent article in aspect 11, in the lower portion of the waist member, the magnitude of force acting during contraction is 50% or more of the force acting during stretching. Compared to the case of being less than 50%, the force during contraction is less likely to become excessively small. Therefore, even when worn in the low-stretched state (50%), in the lower portion, which overlap with the absorbent main body, it is possible to prevent the shortage of the constriction force exhibited by the waist member, making the absorbent main body less likely to shift out of position.

### (Aspect 12)

The underpants-shaped absorbent article according to any one of aspect 1 to 11, wherein in a region located below a center position of the pair of side joining portions in the vertical direction, concerning a magnitude of force when the waist member is contracted in the lateral direction from the most stretched state until the length of the waist member reaches 60% of the most stretched length, and concerning a magnitude of force when the waist member is stretched in the lateral direction from the natural state until the length of the waist member reaches to 60% of the most stretched length, a ratio obtained by dividing the magnitude of force when being contracted by the magnitude of force when being stretched is 60% or more.

According to the underpants-shaped absorbent article in aspect 12, in the lower portion of the waist member, the magnitude of force acting during contraction is 60% or more of the force acting during stretching. Compared to the case of being less than 60%, the force during contraction is less likely to become excessively small. Therefore, even when worn with the stretch ratio of the waist member at 60%, which is slightly higher than 50%, in the lower portion, which overlap with the absorbent main body, it is possible to prevent the shortage of the constriction force exhibited by the waist member, making the absorbent main body less likely to shift out of position.

### (Aspect 13)

The underpants-shaped absorbent article according to any one of aspects 1 to 12, wherein the waist member has a plurality of elastic members between a first sheet and a second sheet that are joined by a plurality of welded portions, the plurality of elastic members being arranged spaced apart in the vertical direction, the plurality of elastic members being capable of stretching and contracting in the lateral direction, and each of the elastic members is attached to the waist member, in a state of being contracted in the lateral direction, by being sandwiched between two of the plurality of welded portions that are adjacent in the vertical direction.

According to the underpants-shaped absorbent article in aspect 13, the waist elastic members are attached to the waist member by the plurality of welded portions that are dispersed, and this make it easier to adjust the stretching force exhibited by the waist member. Also, it is possible to prevent the constriction force from becoming excessively large even in the highly-stretched state.

### Embodiment

As an example of the underpants-shaped absorbent article according to the embodiment of the present invention, an underpants-shaped diaper 1 (hereinafter, simply referred to as "diaper 1") will be described. In addition, the underpants-shaped absorbent article also includes a shorts-type sanitary napkin and an underpants-shaped absorbent pad.

### Basic structure of diaper 1

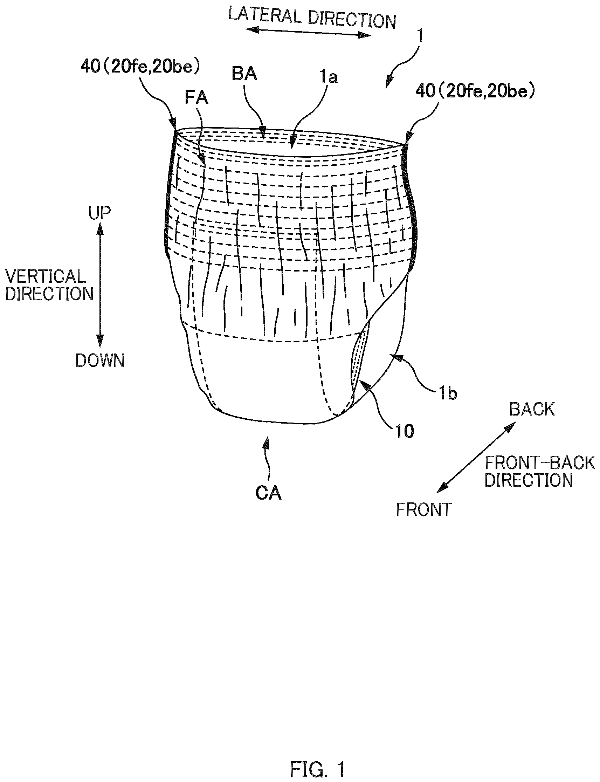

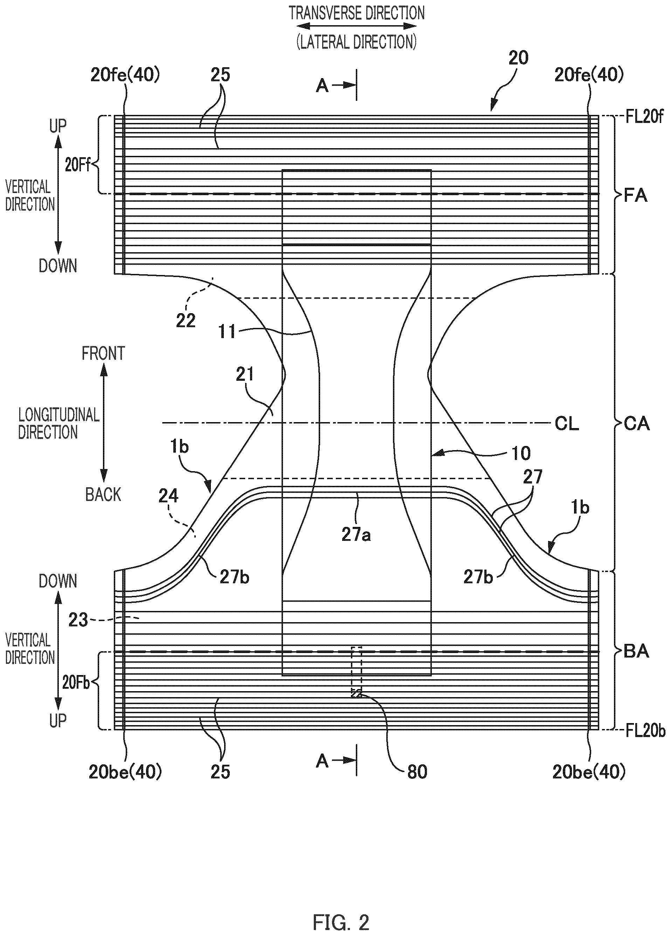

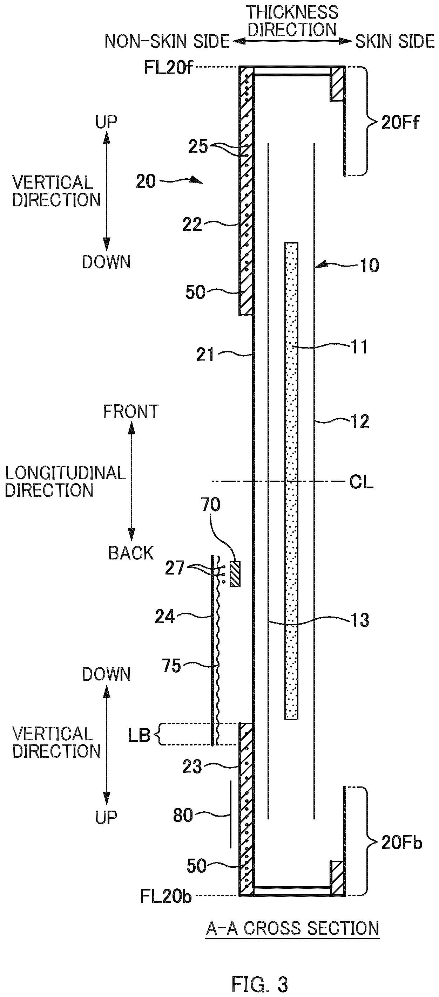

FIG. 1 is a schematic perspective view showing an example of the configuration of the diaper 1 according to the present embodiment. FIG. 2 is a schematic plan view of the diaper 1 in a stretched state as viewed from a skin side in a thickness direction. FIG. 3 is a schematic cross-sectional view taken along line A-A of FIG. 2.

In addition, the "stretched state" of the diaper 1 refers to a state where the entire diaper 1 (entire product) is stretched without wrinkles by stretching the elastic members (e.g., later-described waist elastic members 25 and leg elastic members 27) provided in the diaper 1, or more specifically, a state where the diaper 1 is stretched until the dimensions of the constituent members of the diaper 1 (e.g., a later-described absorbent main body 10 and waist member 20) match or are close to the dimensions of the members on their own. On the other hand, compared to the stretched state, the natural state refers to the state shown in FIG. 1. The "natural state" is a state where the diaper 1 has been left alone for a predetermined period. For example, the waist member 20 of the diaper 1 is pulled outward on both sides in the lateral direction to put the waist member 20 in the "stretched state", and the stretched state is maintained for 15 seconds, and then the diaper 1 is released and placed on a flat surface such as a desk. Then, the state after five minutes of being laid flat on the flat surface is defined as the natural state.

In the underpants-shaped state shown in FIG. 1, the diaper 1 has a "vertical direction", a "lateral direction", and a "front-back direction" that are orthogonal to each other. Also, as shown in FIG. 3, the diaper 1 has a "thickness direction", which is the direction in which members are overlaid on each other. When the wearer wears the diaper 1, the "upper side" in the vertical direction is the side that faces the wearer's torso, and the "lower side" in the vertical direction is the side that faces the wearer's crotch region. Also, in the put-on state, the "front side" in the front-back direction is the side that faces the wearer's abdomen, and the "back side" in the front-back direction is the side that faces the wearer's back. In addition, when the wearer wears the diaper 1, the "skin side" in the thickness direction is the side that comes into contact with the wearer's skin, and the "non-skin side" in the thickness direction is the opposite side.

In addition, in the unfolded state shown in FIG. 2, the diaper 1 has a "longitudinal direction" and a "transverse direction" that are orthogonal to each other. The "longitudinal direction" is a direction conforming to the vertical direction in the underpants-shaped state, and the "transverse direction" is a direction conforming to the lateral direction in the underpants-shaped state.

The diaper 1 of the present embodiment includes: an absorbent main body 10 that has liquid absorbency and absorbs a liquid such as urine; and the waist member 20 that is joined to the non-skin side of the absorbent main body 10 and is arranged at the wearer's waist when the diaper 1 is put on.

### Absorbent main body 10

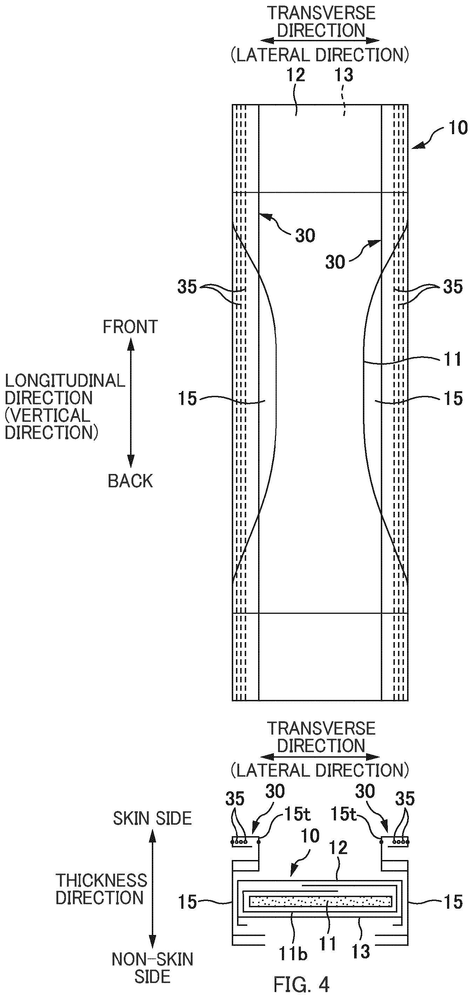

FIG. 4 is a plan view and a cross-sectional view of an absorbent main body 10. As shown in FIG. 2, the absorbent main body 10 of the present embodiment has a substantially rectangular shape in a plan view, and the longitudinal direction (i.e., the long side direction of the absorbent main body 10) conforms to the vertical direction of the diaper 1. As shown in FIGS. 3 and 4, the absorbent main body 10 includes an absorbent core 11 that extends in the longitudinal direction (vertical direction), a top sheet 12 arranged on the skin side with respect to the absorbent core 11, a back sheet 13 arranged on the non-skin side with respect to the absorbent core 11, and a pair of side sheets 15 and 15 arranged on both sides of the absorbent core 11 in the transverse direction (lateral direction).

The absorbent core 11 is a member that absorbs and retains excreted fluid such as urine. For example, the absorbent core 11 can be obtained by molding liquid-absorbent fibers (e.g., pulp) mixed with a superabsorbent polymer (SAP) into a predetermined shape. The absorbent core 11 of the present embodiment is shaped so as to have a substantially hourglass shape with its longitudinal central portion narrowing inward in the lateral direction, as shown in FIG. 4, but the shape of the absorbent core 11 is not limited to this. In addition, the outer circumferential surface of the absorbent core 11 may be covered by a liquid-permeable sheet member (core-wrapping sheet 11b) made of tissue paper or the like.

The top sheet 12 is a liquid-permeable sheet member that can come into contact with the wearer's skin during usage, and is made of hydrophilic air-through nonwoven fabric or the spunbond nonwoven fabric, for example. In the cross-sectional view of FIG. 4, the top sheet 12 is arranged so that its two lateral end portions are folded onto the non-skin side with respect to the absorbent core 11, and therefore the top sheet 12 wraps around and covers the absorbent core 11 from the skin side. In addition, another sheet or the like (not shown) may be provided between the top sheet 12 and the absorbent core 11.

The back sheet 13 is a liquid-impermeable and breathable sheet member (breathable sheet member) for preventing a liquid such as urine absorbed in the absorbent core 11 from leaking to the outside, and is made of a liquid-impermeable film such as polyethylene, polypropylene or the like.

Each of the side sheets 15 is a sheet member located on both transverse sides of the absorbent core 11, and is made of a hydrophobic nonwoven fabric sheet such as a SMS (spunbond-meltblown-spunbond) nonwoven fabric sheet, for example. The vertical (lateral) one-end side of the side sheet 15 is folded so as to be located on the non-skin side with respect to the absorbent core 11 and the back sheet 13. On the other hand, the vertical (lateral) other-end side of the side sheet 15 is folded so as to be located on the skin side with respect to the absorbent core 11 and the top sheet 12, forming a pair of leak-proof wall portions 30 and 30 on both sides of the absorbent main body 10.

The pair of leak-proof wall portions 30 are respectively provided on the two sides of the absorbent main body 10 in the transverse direction (lateral direction), and are portions corresponding to so-called barrier cuffs. When the diaper 1 is put on, the leak-proof wall portions 30 rise toward the wearer's skin on the two sides of the absorbent main body 10 in the lateral direction, thus suppressing the case where excreted fluid such as urine leaks along the wearer's skin to the outside of the absorbent main body 10 in the lateral direction. In addition, each of the leak-proof wall portions 30 may be composed of two or more sheet members (side sheet 15).

As shown in the cross-sectional view of FIG. 4, in the end portion 15t of the portion of the side sheet 15 that is folded toward the skin side in the thickness direction, the side sheet 15 is folded over multiple times so as to be overlaid in the thickness direction, and the leak-proof-wall elastic members 35 are provided between the overlaid portions of the side sheet 15 in a state of being stretched along the longitudinal direction. When the diaper 1 is put on, the end portions 15t of the leak-proof wall portions 30 contracts in the longitudinal direction (vertical direction) due to the stretchability exhibited by the leak-proof-wall elastic members 35, making the absorbent main body 10 likely to fit around the wearer's legs.

### Waist member 20

The waist member 20 is a sheet member located on the non-skin side of the absorbent main body 10, and has a skin-side sheet 21, non-skin-side sheets 22 and 23, and a cover sheet 24. Also, as shown in FIG. 2, the portion of the waist member 20 that overlaps the later-described side joining portion 40 provided on the front side in the longitudinal direction with respect to the center position CL (on the front side in the front-back direction in FIG. 1) is referred to as a "front waist portion FA". Similarly, the portion of the waist member 20 that overlaps the side joining portion 40 provided on the back side in the longitudinal direction with respect to the center position CL (on the back side in the front-back direction in FIG. 1) is referred to as a "back waist portion BA". In the longitudinal direction, the portion between the back end of the front waist portion FA and the front end of the back waist portion BA is referred to as a "crotch portion CA".

The skin-side sheet 21 is a sheet member that is arranged on the skin side of the waist member 20, and is made of spunbond nonwoven fabric, SMS nonwoven fabric, or the like, for example. As shown in FIG. 2, the skin-side sheet 21 extends continuously from the front side to the back side in the longitudinal direction, and has a shape narrowed inward in the lateral direction in the crotch portion CA. This narrowed portion becomes the leg openings 1b (described later) in the diaper 1 in the underpants-shaped state.

The non-skin-side sheet 22 is a sheet member overlaid on the non-skin side of the skin-side sheet 21, on the front side in the longitudinal direction. Similarly to the skin-side sheet 21, the non-skin-side sheet 22 is made of spunbond nonwoven fabric, SMS nonwoven fabric, or the like. The skin-side sheet 21 and the non-skin-side sheet 22 are overlaid in the thickness direction and joined to each other by a plurality of welded portions 50, 50, ..., which will be described later. In addition, between the skin-side sheet 21 and the non-skin-side sheet 22, a plurality of the waist elastic members 25, 25, ... are provided. The plurality of waist elastic members 25, 25, ... are arranged side-by-side spaced apart in the longitudinal direction (vertical direction), and are attached between the skin-side sheet 21 and the non-skin-side sheet 22 in a state of being stretched in the transverse direction (lateral direction). As a result, the waist elastic members 25 exhibit the stretchability along the transverse direction (lateral direction), which then gives the stretchability to the front waist portion FA of the waist member 20.

The waist elastic members 25 can be made of, for example, an elastic resin material whose main component is thermoplastic elastic resin, which is molded into the shape of an elastic string. As the above-mentioned thermoplastic elastic resin, a thermoplastic resin that exhibits rubber elasticity at room temperature is preferable, for example, and an example thereof is thermoplastic elastomers described in JIS K 6418:2007. As the above-mentioned thermoplastic elastic resin, a material with a temperature range in which elastic deformation occurs of approximately 100°C or less is preferable. Examples of the above-mentioned thermoplastic elastic resin include olefin elastomers (e.g., VERSIFY (trademark) from The Dow Chemical Company), propylene elastomers (e.g., Vistamaxx (trademark) from Exxon Mobil Corporation), and styrene elastomers (e.g., Quintac (trademark) from Zeon Corporation).

The non-skin-side sheet 23 is a sheet member overlaid on the non-skin side of the skin-side sheet 21, on the back side in the longitudinal direction, and has a similar configuration to the non-skin-side sheet 22. That is, the non-skin-side sheet 23 is joined to the skin-side sheet 21 by the plurality of welded portions 50. In addition, between the skin-side sheet 21 and the non-skin-side sheet 23, a plurality of the waist elastic members 25, 25, ... are provided in a manner of being capable of stretching/contracting along the transverse direction (lateral direction).

The cover sheet 24 is a sheet member overlaid on the non-skin side of the skin-side sheet 21, similarly to the non-skin-side sheet 23, on the back side in the longitudinal direction. As shown in FIG. 3, in the diaper 1, the cover sheet 24 is located below the non-skin-side sheet 23 in the vertical direction (on the front side in the longitudinal direction), and a part thereof is arranged overlapping the non-skin-side sheet 23. Hereinafter, the portion where the cover sheet 24 and the non-skin-side sheet 23 overlap with respect to the vertical direction (longitudinal direction) is also referred to as a "back overlapping portion LB".

The cover sheet 24 is joined to a part of the skin-side sheet 21 and a part of the non-skin-side sheet 23 via the adhesive surface 75, which is made of an adhesive such as a hot-melt adhesive or the like. Between the cover sheet 24 and the skin-side sheet 21 in the thickness direction, there are provided the leg elastic members 27, which are made of elastic strings or the like (see FIG. 3). The leg elastic members 27 are attached to the waist member 20 through an adhesive portion 70, which is made up of an adhesive such as hot-melt adhesive or the like applied to a certain area of the non-skin-side surface of the skin-side sheet 21. In the present embodiment, the leg elastic members 27 each have a straight portion 27a in the transverse (lateral) center and curved portions 27b on the two sides in the transverse direction. In the straight portion 27a, the portion where the leg elastic member 27 overlaps the absorbent main body 10 (absorbent core 11) is arranged in a straight line along the transverse direction. In the curved portion 27b, the leg elastic member 27 is arranged in a curved manner along the contour of the leg opening 1b. Since substantially the entire area of the leg elastic member 27 is fixed by the adhesive portion 70, it is possible to maintain the shapes of the straight portion 27a and the curved portions 27b of the leg elastic member 27. The stretchability exhibited by the leg elastic members 27 makes the leg openings 1b likely to fit around the wearer's legs on the back side when the diaper 1 is put on, and also makes the portion of the waist member 20 covering the wearer's buttocks less likely to roll up.

In addition, in the upper end portion of the waist member 20 in the vertical direction, a part of the skin-side sheet 21 and parts of the non-skin-side sheet 22 and 23 are folded downward. In FIG. 3, the skin-side sheet 21 and the non-skin-side sheet 22 form a front fold-back portion 20Ff, which is a portion folded back downward in the vertical direction and toward the skin side in the thickness direction, at the front bending position FL20f located in the front upper end portion of the waist member 20. The front fold-back portion 20Ff is joined to at least portions of sheet members located adjacent in the thickness direction (the skin-side sheet 21 and the absorbent main body 10, the top sheet 12 in FIG. 3) with an adhesive such as a hot-melt adhesive.

Similarly, in FIG. 3, the skin-side sheet 21 and the non-skin-side sheet 23 form a back fold-back portion 20Fb, which is a portion folded back downward in the vertical direction (toward the front side in the longitudinal direction) and toward the skin side in the thickness direction, at the back bending position FL20b located in the back upper end portion of the waist member 20. The back fold-back portion 20Fb is joined to at least portions of sheet members adjacent in the thickness direction (the skin-side sheet 21 and the absorbent main body 10, the top sheet 12 in FIG. 3) with an adhesive such as a hot-melt adhesive.

In the diaper 1, the front fold-back portion 20Ff may be formed by folding back only either sheet member of the skin-side sheet 21 or the non-skin-side sheet 22, at the front bending position FL20f. Similarly, the back fold-back portion 20Fb may be formed by folding back only either sheet member of the skin-side sheet 21 or the non-skin-side sheet 23, at the back bending position FL20b. That is, the fold-back portions 20Ff and 20Fb each may be formed by one sheet member.

Also, a separate sheet member may be provided on the skin side with respect to the fold-back portions 20Ff and 20Fb. For example, by arranging this sheet member so that it straddles the vertical lower ends of the fold-back portions 20Ff and 20Fb in the vertical direction from the skin side (not shown in FIG. 3), it is possible to prevent the lower end edges of the fold-back portions 20Ff and 20Fb from digging into the wearer's skin when the diaper 1 is put on.

The diaper 1 is folded one time from the unfolded state shown in FIG. 2, and at this stage, the absorbent main body 10 and the waist member 20 is folded in the longitudinal direction at a bending position, which is the longitudinal center position CL (shown as the single-dotted chain line in FIG. 2). In this folded state, of the waist member 20, the front waist portion FA and the back waist portion BA are overlaid on each other in the front-back direction. The transverse side portions 20fe and 20fe of the front waist portion FA and the transverse side portions 20be and 20be of the back waist portion BA are joined together using a known joining means such as seal welding, thus forming a pair of the side joining portions 40 and 40. As a result, the two portions of the folded waist member 20 are connected on the front side and the back side to form a ring, thus forming one waist opening 1a and the pair of leg openings 1b as shown in FIG. 1, and obtaining the underpants-shaped diaper 1.

### Attaching of waist elastic members 25

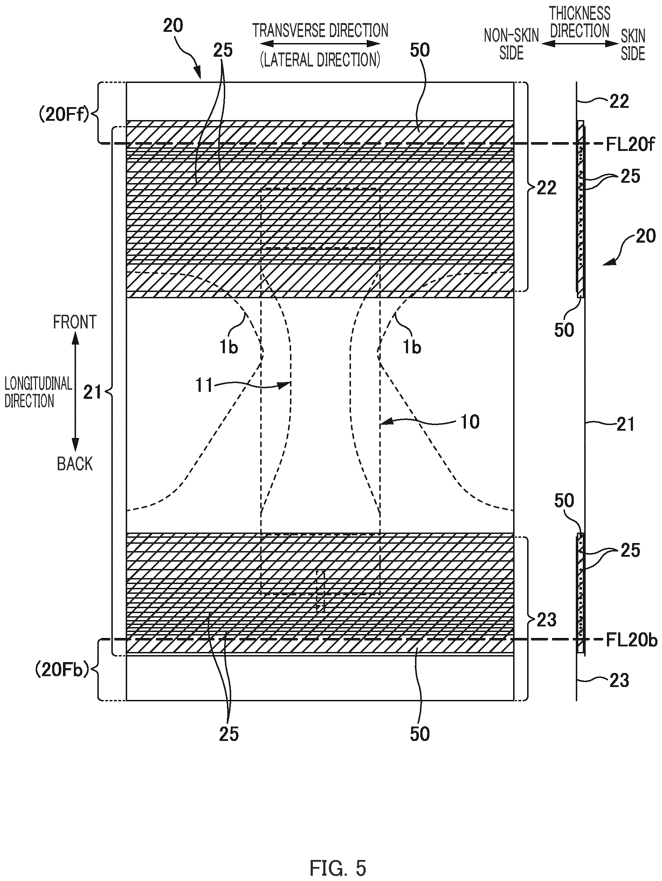

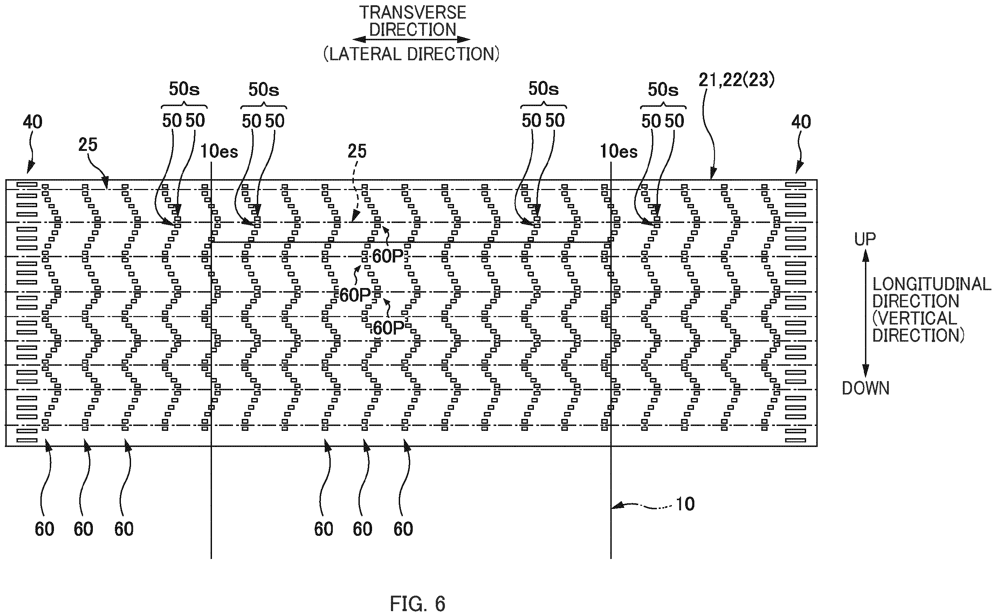

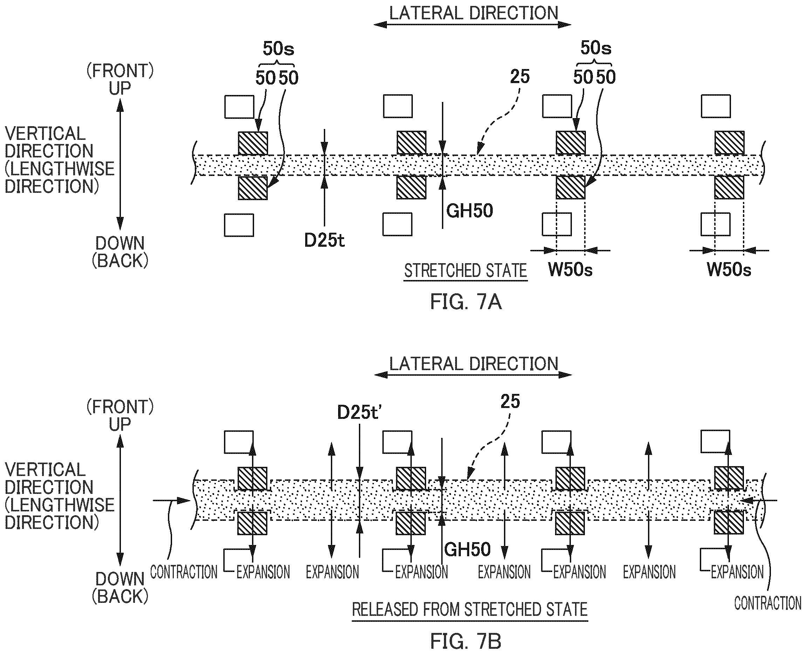

The specific method for attaching the waist elastic members 25 and the leg elastic members 27 to the waist member 20 will be explained. First, the method for attaching the waist elastic members 25 will be described. FIG. 5 is a diagram illustrating the range in the waist member 20 within which a plurality of the welded portions 50 are formed. FIG. 6 is a diagram illustrating an example of the arrangement of the welded portions 50. FIGS. 7A and 7B are diagrams illustrating the principle by which the waist elastic members 25 are attached to the waist member 20 by the welded portions 50.

In the waist member 20 of the diaper 1, the skin-side sheet 21 and the non-skin-side sheets 22 and 23 are joined by the plurality of welded portions 50, 50, ... that are formed by using a known welding means such as ultrasonic welding. In FIG. 5, the hatched portion is the region where the welded portions 50 are formed when the non-skin-side sheets 22 and 23 (corresponding to the second sheet) are joined to the non-skin side of the skin-side sheet 21 (corresponding to the first sheet) during the manufacturing process of diaper 1. At the stage of joining the skin-side sheet 21 and the non-skin-side sheets 22 and 23, the leg openings 1b have not yet been formed (the sheet member has not yet been cut). In addition, in FIG. 5, the position of the absorbent main body 10 (absorbent core 11) is indicated by a dotted line, but at the stage of forming the welded portions 50, the absorbent main body 10 has not yet been joined to the waist member 20.

As shown in FIG. 5, the skin-side sheet 21 and the non-skin-side sheets 22 and 23 are joined in the portions where they overlap in the thickness direction in the stretched state before being folded at the front bending position FL 20f and the back bending position FL 20b. That is, the welded portions 50 are formed in ranges respectively straddling the bending positions FL20f and FL20b in the front-back direction. In the range indicated by the hatched portions in FIG. 5, between the skin-side sheet 21 (first sheet) and the non-skin-side sheets 22 and 23 (second sheet) that face each other in the thickness direction, the waist elastic members 25, 25, ... which can stretch/contract along the lateral direction are attached due to the welded portions 50, side-by-side spaced apart in the vertical direction (longitudinal direction).

In the waist member 20 of the present embodiment, as shown in FIG. 6, the plurality of welded portions 50, 50, ... are arranged side-by-side spaced apart in the vertical direction (longitudinal direction), thereby forming a welded portion row 60 extending along the vertical direction. A plurality of the welded portion rows 60 are provided spaced apart in the lateral direction (transverse direction). In FIG. 6, for the sake of simplicity, the arrangement and quantities of the waist elastic members 25 and the welded portions 50 are shown schematically. Each welded portion row 60 is arranged so as to have convex portions 60P that are convex on the one side or the other side in the lateral direction. That is, since the lateral positions of the plurality of welded portions 50 included in the welded portion row 60 are different from one another, the welded portion row 60 is formed to have projections and recesses as a whole. In the example of FIG. 6, the welded portion row 60 is formed extending from top to bottom in the vertical direction so as to meander leftward and rightward. However, the arrangement of each of the welded portions 50 is not limited to the example of FIG. 6, and the welded portion row 60 may have convex portions 60P only on the one side in the lateral direction. Also, the welded portion row 60 may be arranged in a straight line along the vertical direction without having any bumps in the lateral direction.

Of the plurality of welded portions 50, 50, ... included in the welded portion row 60, the waist elastic member 25 is sandwiched above and below between the two welded portions 50 and 50 adjacent in the vertical direction, thereby attaching the waist elastic member 25 to the waist member 20. That is, the pair of welded portions 50 and 50 arranged on both vertical sides of the waist elastic member 25 form a welded portion pair 50s, and the waist elastic member 25 is attached by the welded portion pair 50s.

As shown in FIG. 7A, a pair of the welded portions 50 and 50 that form the welded portion pair 50s are arranged with a gap GH50 therebetween in the vertical direction. The size of the gap GH50 is set to be equal to or slightly larger than the outer diameter D25t of the waist elastic member 25 (e.g., an elastic string) in a state of being stretched to a target stretch factor in the lateral direction (GH50 ≥ D25t). That is, the waist elastic member 25 in the stretched state is placed between the welded portion pair 50s in the vertical direction. With such a configuration, in the manufacturing process of the diaper 1, the waist elastic members 25 in the stretched state are placed between the skin-side sheet 21 and the non-skin-side sheet 22 (23), and then when the skin-side sheet 21 and the non-skin-side sheet 22 (23) are joined, it is possible to form the welded portions 50 without overlapping the waist elastic members 25. Here, the "stretch factor" refers to the value R (= L1/L0) that indicates the ratio of the total length L1 of the stretched waist elastic member 25 to the total length L0 in a natural no-load state.

Then, when the waist elastic member 25 is relaxed from the stretched state, as shown in FIG. 7B, the waist elastic member 25 expands in the vertical direction while contracting in the lateral direction, and the outer diameter D25t' after expansion becomes larger than the gap GH50 of the welded portion pair 50s in the vertical direction (D25t' > GH50). Therefore, the expansion of the waist elastic member 25 in the vertical direction is restricted between the welded portion pair 50s, and as a result, the waist elastic member 25 is substantially sandwiched between the welded portions 50 and 50 in the vertical direction. As a result, the welded portion pair 50s restricts the positions of the waist elastic member 25 in the vertical direction and the lateral direction. Accordingly the waist elastic member 25 is attached to the waist member 20 in a manner of being capable of stretching/contracting, and the waist member 20 is given the stretchability in the lateral direction.

The skin-side sheet 21 (first sheet) and the non-skin-side sheet 22, 23 (second sheets) are joined by the welded portions 50, which are made by joining means using ultrasonic welding or the like, and therefore the joining strength of the waist member 20 can be securely secured. In addition, since the welded portions are located in a dispersed manner, the flexibility of the waist member 20 is more likely to be maintained, and the texture can be made good.

In addition, in the diaper 1 in the underpants-shaped state, the waist elastic member 25 is joined by the side joining portions 40 in the two lateral end portions of the waist member 20, and therefore even if the waist member 20 is stretched in the lateral direction when the diaper 1 is put on, the waist elastic member 25 does not come off the waist member 20.

### Constriction force when putting on diaper 1

When putting on an underpants-shaped absorbent article such as the diaper 1, the wearer's legs are passed through the pair of leg openings 1b and 1b, and the waist member 20 and the absorbent main body 10 are pulled up toward the crotch side to fit the wearer's body. At this time, the waist member 20 is in a state of being stretched in the lateral direction from the natural state. By generating the constriction force along the circumferential direction of the waist opening 1a due to the stretchability exhibited by the waist elastic members 25, the waist member 20 fits around the wearer's waist.

Incidentally, in a typical underpants-shaped diaper, when being worn by the wearer, the length of the waist member in the lateral direction (circumference of the waist opening) is not necessarily always constant. For example, when the wearer moves or breathes, the circumference of the waist changes, and therefore the waist member stretches or contracts in the lateral direction in response thereto. In conventional underpants-shaped diapers, accompanied with the stretching/contracting of the waist member in the lateral direction, the constriction force acting around the wearer's waist may become larger, causing a risk of making the wearer feel pressure or discomfort. In particular, when an underpants-shaped diaper is worn with being in the highly-stretched state, in which the length of the waist member in the lateral direction is approximately 80% of its maximum stretched length (corresponding to the stretched state mentioned above), the constriction force becomes larger accompanied with the stretching/contracting of the waist member, making it difficult to wear comfortably.

In contrast, in the diaper 1 of the present embodiment, the waist elastic members 25 are attached to the waist member 20 by the plurality of welded portions 50 that are dispersed, thereby adjusting the stretching force exhibited by the waist member 20 and preventing the constriction force from becoming excessively large even in the highly-stretched state. The following describes the results of tests of the magnitude of the constriction force exhibited by the waist member 20, on the diaper 1 of the present embodiment and conventional underpants-shaped diapers A to F as Comparative Examples.

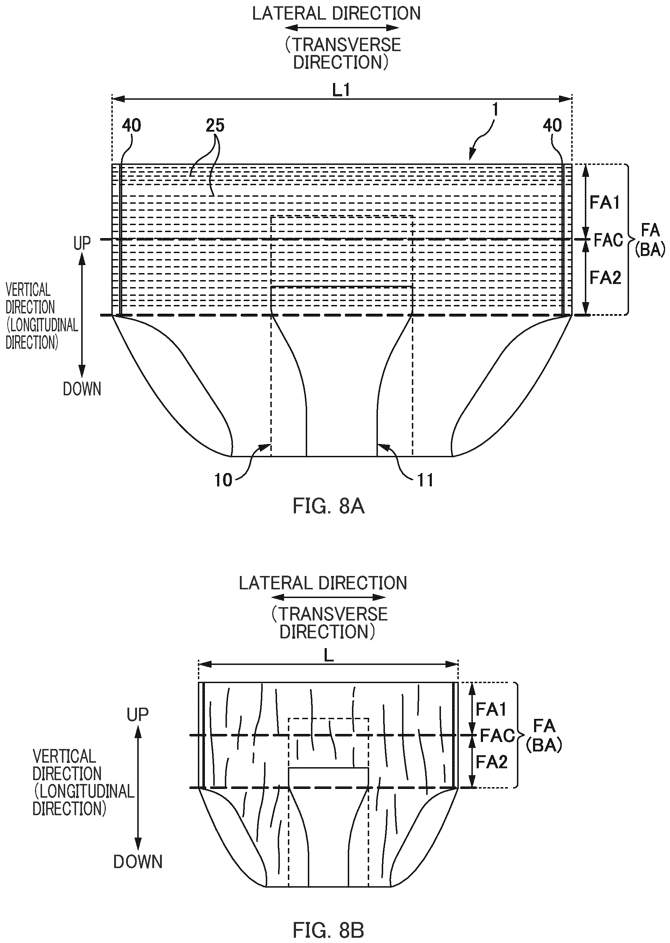

FIG. 8 is a diagram illustrating the concept of a tensile test for measuring the magnitude of the constriction force of the waist member 20. First, both ends of the waist member 20 of the diaper 1 in the natural state are pulled in the lateral direction, to reach the stretched state as shown in FIG. 8A (i.e., the state in which the dimensions of the waist member 20 in the lateral direction are stretched to match the dimensions of the constituent members on their own). In the following, for clarity of explanation, the state of FIG. 8A is also referred to as a "maximum stretched state". In other words, the "maximum stretched state" is the state when the waist member 20 has stretched the most in the lateral direction. The length, in the lateral direction, of the waist member 20 in the maximum stretched state is defined as L1.

Next, after allowing the diaper 1 to return to be in the natural state, the two ends of the waist member 20 of the diaper 1 (e.g., the positions of the side joining portions 40) are clamped by the chucks of a known tensile tester (e.g., a universal testing machine manufactured by Instron Co.). At this time, the distance between the chucks of the tensile tester is set to 180 mm. Furthermore, the distance between the chucks, 180 mm, is less than the lateral length of the waist member 20 in the natural state. From this state (i.e., the natural state), the waist member 20 is pulled toward the two lateral sides at a pulling speed of 300 mm/min, and is stretched until the waist member 20 becomes in the maximum stretched state. Subsequently, the waist member 20 in the maximum stretched state is contracted at a rate of 300 mm/min until its lateral length returns to 180 mm. This cycle is repeated twice. Then, during the first cycle of stretching, the magnitude of force (N) when the waist member 20 is stretched until its lateral length reaches a predetermined length L (see FIG. 8B) is measured and recorded. Also, during the second cycle of contracting, the magnitude of force (N) when the waist member 20 is contracted until its lateral length reaches a predetermined length L (see FIG. 8B) is measured and recorded.

In this way, the magnitude of force when the waist member 20 is stretched until its lateral length reaches to (L/L1)% of the maximum stretched state and the magnitude of force when it is contracted until its lateral length reaches to (L/L1)% of the maximum stretched state are measured, and the magnitude of the constriction force due to the stretching and contraction of the waist member 20 is evaluated. As comparative examples, commercially available conventional underpants-shaped diapers A to F were also measured under the same conditions and compared with the diaper 1.

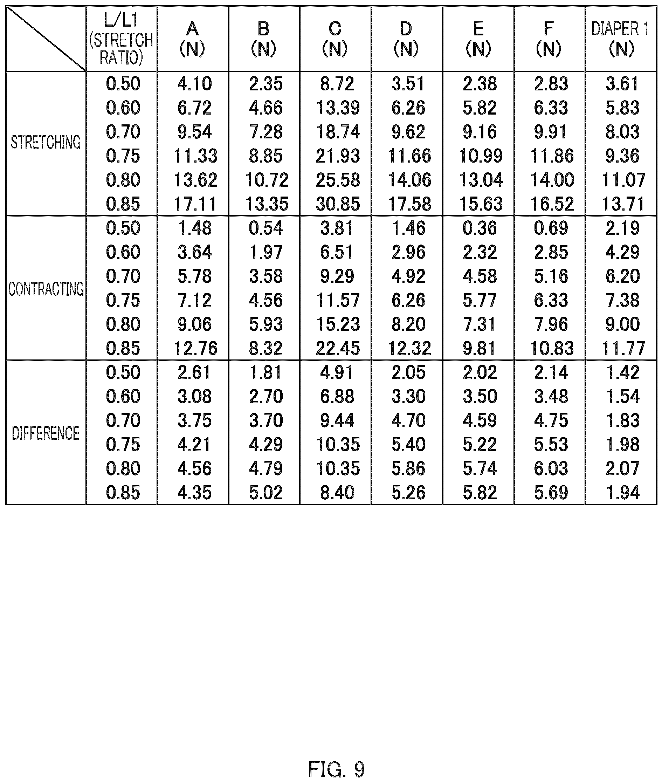

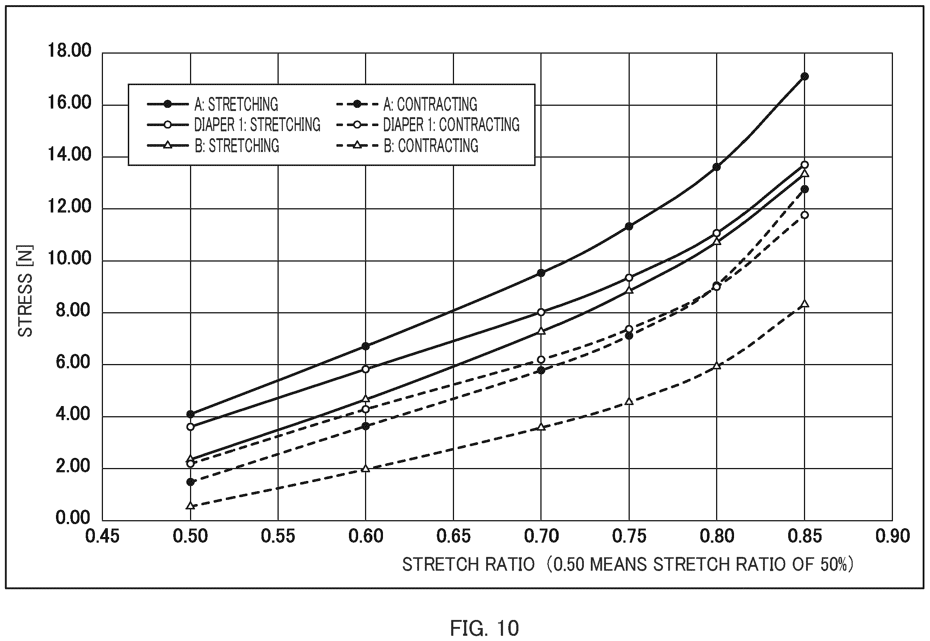

FIG. 9 is a table showing the results of the tensile test on the diaper 1 and Comparative Examples A to F. FIG. 10 is a graph showing the relationship between the stretch ratio of the waist member and the magnitude of force during stretching/contracting for the diaper 1 and Comparative Examples A and B. In FIG. 9, the magnitude of force (N) when the waist member 20 is stretched or contracted to the lateral length L of the waist member 20 relative to the lateral length L1 of the waist member 20 that is in the maximum stretched state is displayed for each stretch ratio of the waist member 20 (L/L1). For example, the force required to stretch, in the lateral direction, the waist member 20 of the diaper 1 from the natural state until L/L1 = 0.5 (i.e. the stretch ratio is 50%) is 3.61N. In addition, the force required to contract, in the lateral direction, the waist member 20 of the diaper 1 from the maximum stretched state until L/L1 = 0.5 (the stretch ratio is 50%) is 2.19 N.

The range of the stretch ratio shown in FIG. 9 (0.5 to 0.85) is the expected stretch range of the waist member in the cases of wearing the diaper 1 and Comparative Examples A to F. That is, in the normal use of the diaper 1 and the like, the waist member 20 stretches or contracts in the lateral direction within the range where the stretch ratio is 0.5 to 0.85. In the following, the range of 0.5 to 0.6 (50% to 60%) where the stretch ratio of the waist member 20 is relatively low is referred to as a "low-stretched state," and the range of 0.75 to 0.85 (75% to 85%) where the stretch ratio is relatively high is referred to as a "highly-stretched state ".

In FIG. 9, the magnitude of force when the waist member 20 of the diaper 1 is stretched from the natural state to the stretch ratio of 0.80 (80% of the most stretched length) is 11.07 N. On the other hand, the magnitude of force when the waist member 20 is contracted from the maximum stretched state to the stretch ratio of 0.80 is 9.00 N. This means that if the diaper 1 is being worn in the highly-stretched state, where the stretch ratio of the waist member 20 is approximately 0.80, when the waist member 20 stretches in the lateral direction due to the wearer's body movement or the like, a force of 11.07 N is exerted, and when the waist member 20 contracts in the lateral direction, a force of 9.00 N is exerted. And, the difference in the magnitude of force between stretching and contraction, 2.07 N (= 11.07 - 9.00), is the constriction force which is exerted on the wearer's waist accompanied with the stretching/contracting of the waist member 20.

In contrast, in the underpants-shaped diapers of Comparative Examples A to F, the constriction force was larger than the diaper 1 under the same conditions (the stretch ratio 0.80). For example, in Comparative Example A, the difference in the magnitude of force between when stretched and when contracted at the stretch ratio 0.8 (80%) was 4.56 N (=13.62 - 9.06), which is clearly a larger force than that acting on the diaper 1 (see FIG. 10). Similarly, in Comparative Example B, the difference in the magnitude of force between when stretched and when contracted at the stretch ratio 0.8 was 4.79 N (=10.72 - 5.93), which is a larger force than that acting on the diaper 1 (see FIG. 10). Thus, in any of comparative examples A to F, in the case of the highly-stretched state where the stretch ratio is about 0.80, the constriction force, which is larger than 4.0 N, acts around the wearer's waist (see FIGS. 9 and 10).

In the diaper 1 of the present embodiment, when the waist member 20 is worn in the highly-stretched state (80%), when the waist member stretches or contracts in the lateral direction due to the wearer's body movement or the like, the difference in the magnitude of force between when being stretched and when being contracted is 4.0 N or less. The constriction force acting around the wearer's waist is smaller than when the difference in the magnitude of force is greater than 4.0 N. In other words, the diaper 1 of the present embodiment can be worn "comfortably" even in the highly-stretched state.

In addition, actually, when the waist member 20 of the diaper 1 is worn in the highly-stretched state (80%), whether the wearer feels "comfortably" can be evaluated by conducting a sensory test. For example, when 10 subjects are asked to perform a specified movement (walking, etc.) while wearing the diaper 1 in the highly-stretched state (80%), if the proportion of subjects who feel the unpleasantness or the discomfort is two or less, then diaper 1 is deemed to be worn "comfortably.".

Furthermore, the diaper 1 of the present embodiment can be worn "comfortably" even when worn in the low-stretched state, the stretch ratio of the waist member 20 is about 0.60. Specifically, in the diaper 1, the magnitude of force when the waist member 20 is stretched from the natural state to the stretch ratio of 0.60 (60% of the most stretched length) is 5.83 N. On the other hand, the magnitude of force when the waist member 20 is contracted from the maximum stretched state to the stretch ratio of 0.60 is 4.29 N. That is, when the diaper 1 is being worn in the low-stretched state, where the stretch ratio of the waist member 20 is 60%, the difference in the magnitude of force between stretching and contraction is 1.54 N (= 5.83 - 4.29) (see FIG. 9) .

In contrast, in the Comparative Examples A to F, when the diaper was worn with the stretch ratio = 0.60 (the low-stretched state), the difference in the magnitude of force between stretching and contraction was greater than 2.50 N in all cases (see FIG. 9), indicating that the constriction force, which is greater than that of the diaper 1, was exerted. In other words, in the diaper 1, when the waist member 20 is worn in the low-stretched state (60%), the difference in the magnitude of force between when being stretched and when being contracted is 2.5 N or less. Therefore the constriction force is weaker than when the difference in the magnitude of force is greater than 2.5 N, making it possible to wear the diaper comfortably even in the low-stretched state.

Furthermore, the diaper 1 can be worn "comfortably" even when worn in the maximum stretched state expected in normal use (the stretch ratio of the waist member 20 is 0.85). Specifically, in the diaper 1, the magnitude of force when the waist member 20 is stretched from the natural state to the stretch ratio of 0.85 (85% of the maximum stretched length) is 13.71 N. On the other hand, the magnitude of force when the waist member 20 is contracted from the maximum stretched state to the stretch ratio of 0.85 is 11.77 N. That is, when the diaper 1 is being worn in the highly-stretched state, where the stretch ratio of the waist member 20 is 85%, the difference in the magnitude of force between stretching and contraction is 1.94 N (= 13.71 - 11.77).

In contrast, in the Comparative Examples A to F, when the diaper was worn with the stretch ratio = 0.85 (the highly-stretched state), the difference in the magnitude of force between stretching and contraction was greater than 4.0 N in all cases (see FIG. 9), indicating that the constriction force, which is greater than that of the diaper 1, was exerted. In other words, in the diaper 1, when the waist member 20 is worn in the highly-stretched state (85%), the difference in the magnitude of force between when being stretched and when being contracted is 4.0 N or less. Therefore, the constriction force is weaker than when the difference in the magnitude of force is greater than 4.0 N, making it possible to wear the diaper comfortably even in the maximum stretched state which can be expected.

As described above, throughout the low-stretched state to the highly-stretched state, the diaper 1 of the present embodiment does not become excessively tight compared to conventional underpants-shaped diapers, making it possible to wear the diaper 1 comfortably. However, in the underpants-shaped diapers, if the waist member is excessively loose, the waist member becomes easier to shift out of place, causing a risk that the fit deteriorates. In particular, if the diaper 1 is worn in the low-stretched state, which the stretch ratio of the waist member is about 0.5 to 0.6, there is a risk that the constriction force by the waist member become excessively weak.

For example, in Comparative Example C, the magnitude of force when the waist member is stretched from the natural state to the stretch ratio of 0.50 (50% of the most stretched length) is 8.72 N, and the magnitude of force when the waist member is contracted from the maximum stretched state to the stretch ratio of 0.50 is 3.81 N. When worn in the low-stretched state (50%), the value obtained by dividing the force when being contracted by the force when being stretched is 43.7% (= 3.81/8.72). In other words, the magnitude of force that is exerted when contracting is half or less of the force that is exerted when stretching. In this case, the force during contracting become excessively small, and the constriction force for the waist portion becomes insufficient, causing a risk of make the diaper more likely to shift out of place. Furthermore, in all of the Comparative Examples A to F, the value obtained by dividing the force during contracting by the force during stretching is 43.7% or less (less than 50%) . This causes a risk that the diaper in the low-stretched state (50%) is likely to shift out of place during contracting.

On the other hand, in the diaper 1 of the present embodiment, the magnitude of force when the waist member 20 is stretched from the natural state to the stretch ratio of 0.50 is 3.61 N, and the magnitude of force when it is contracted from the maximum stretched state to the stretch ratio of 0.50 is 2.19 N. When being worn in the low-stretched state (50%), the value obtained by dividing the force during contracting by the force during stretching is 60.7% (= 2.19/3.61). In other words, the magnitude of force acting when the waist member 20 contracts is 50% or more of the magnitude of force acting when the waist member 20 stretches. The force acting during contracting is less likely to be excessively small compared to the case of being less than 50%. Therefore, even if the diaper 1 is worn in the low-stretched state (50%), the waist member 20 is less likely to shift out of place.

Furthermore, if the stretch ratio of the waist member 20 is set to 0.6, which is slightly higher than 0.5, in Comparison Example A, the magnitude of force when the waist member is stretched from the natural state until the stretch ratio reaches 0.60 is 6.72 N, and the magnitude of force when it is contracted from the maximum stretched state until the stretch ratio reaches 0.60 is 3.64 N. The value obtained by dividing the force during contracting by the force during stretching is 54.2% (= 3.64/6.72). In addition, in all of the Comparative Examples A to F, the value obtained by dividing the force during contracting by the force during stretching is 54.2% or less (less than 60%).

On the other hand, in the diaper 1 of the present embodiment, the magnitude of force when the waist member 20 is stretched from the natural state to the stretch ratio of 0.60 is 5.83 N, and the magnitude of force when it is contracted from the maximum stretched state to the stretch ratio of 0.60 is 4.29 N. The value obtained by dividing the force during contracting by the force during stretching is 73.6% (= 4.29/5.83). In other words, the magnitude of force acting when the waist member 20 contracts is 60% or more of the magnitude of force acting when the waist member 20 stretches. The force acting during contracting is less likely to be excessively small compared to the case of being less than 60%. Therefore, even if the diaper 1 is worn in the low-stretched state (60%), the waist member 20 is less likely to shift out of place.

In addition, when putting on the diaper 1, the waist member 20 is pulled to both lateral sides to spread out the waist opening 1a and the leg openings 1b, and then the wearer is pulled up from the toe side to the crotch side. At this time, the waist member 20 is stretched to become from the low-stretched state to the highly-stretched state. In the diaper 1, the rate of increase in force when the waist member 20 is significantly stretched is restricted to a predetermined value or less, thereby preventing the constriction force caused by the waist member 20 from becoming excessively large.

In diaper 1, when the waist member 20 in the natural state is being stretched in the lateral direction, the rate of increase in force when stretched from 50% of the maximum stretched state to 85% is 28.85 N (= (13.71 - 3.61)/(0.85 - 0.50)). That is, in FIG. 10, the slope when the stretch ratio of the diaper 1 changes from 0.5 to 0.85 is 28.85 N (less than 30 N).

On the other hand, in Comparative Example B, when the waist member is being stretched in the lateral direction, the rate of increase in force when stretched from 50% of the maximum stretched state to 85% is 31.41 N (= (13.35 - 2.35)/(0.85 - 0.50)). In addition, in all of the Comparative Examples A to F, the rate of increase in force when stretched from 50% of the maximum stretched state to 85% is greater than 30 N, which is greater than that of the diaper 1. In other words, in the diaper 1, the rate of increase in force when the waist member 20 is stretched in the range of the stretch ratio 0.5 to 0.85 is 30 N or less, making the waist member 20 more likely to stretch gradually compared to the case where the rate is greater than 30 N. This prevents a sudden increase in the constriction force and makes it easier to wear diaper 1 comfortably.

In addition, the rate of increase in force when the waist member 20 of the diaper 1 is stretched in the range of the stretch ratio 0.75 to 0.85 (i.e., the highly-stretched state) is restricted to a specified value or less, thereby preventing the constriction from excessively large even in the highly-stretched state. Specifically, when the waist member 20 is being stretched in the lateral direction, the rate of increase in force when stretched from 75% of the maximum stretched state to 85% is 43.5 N (= (13.71 - 9.36)/(0.85 - 0.75)). That is, in FIG. 10, the slope when the stretch ratio of the diaper 1 changes from 0.75 to 0.85 is 43.52 N (less than 44 N).

On the other hand, in Comparative Example B, when the waist member is being stretched in the lateral direction, the rate of increase in force when stretched from 75% of the maximum stretched state to 85% is 45.0 N (= (13.35 - 8.85)/(0.85 - 0.75)). In addition, in all of the Comparative Examples A to F, the rate of increase in force when stretched from 75% of the maximum stretched state to 85% is greater than 44 N, which is greater than that of the diaper 1. In other words, in the diaper 1, the rate of increase in force when the waist member 20 is stretched in the range of the stretch ratio 0.75 to 0.85 is 44 N or less, and compared to the case where the rate of iincrease in force is greater than 44 N, it is possible to wear the diaper 1 comfortably even in the highly-stretched state.

In addition, when the waist member 20 of diapers 1 was divided into two halves in the vertical direction, the tensile test is carried out for the upper portion and the lower portion in the same manner as described above. Specifically, of the front waist portion FA (the back waist portion BA) shown in FIG. 8, in the vertical direction, the portion above the center position FAC (corresponding to the center position of the side joining portion 40) is referred to as an upper portion FA1, and the portion below the center position FAC is referred to as a lower portion FA2. Then, the waist member 20 was cut along the lateral direction at the center position FAC in the vertical direction, and divided into the upper portion FA1 and the lower portion FA2, and the tensile test was performed on each of them in the same manner as described above.

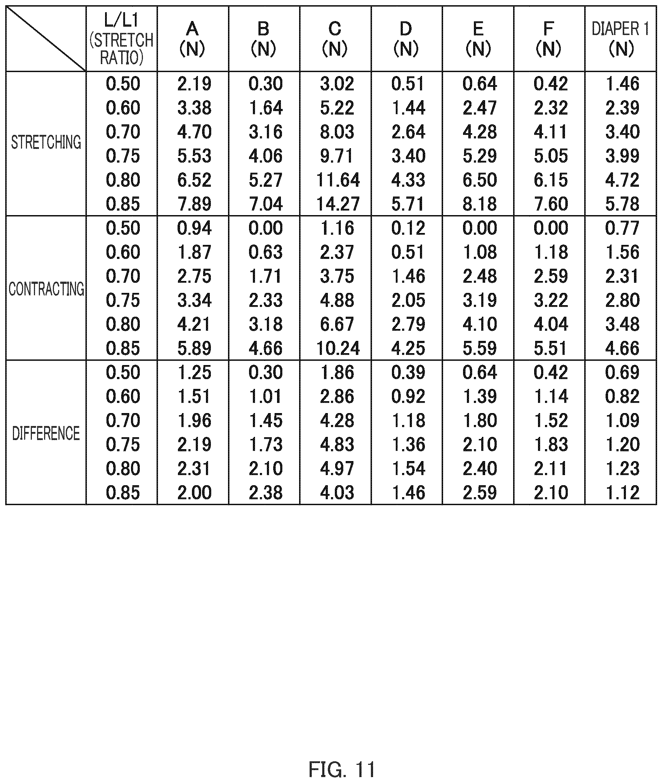

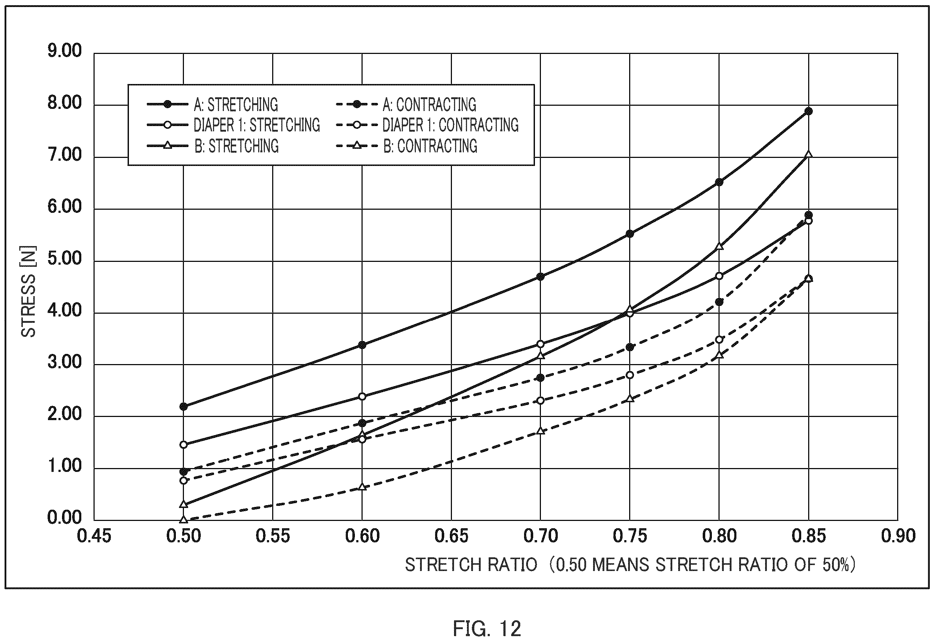

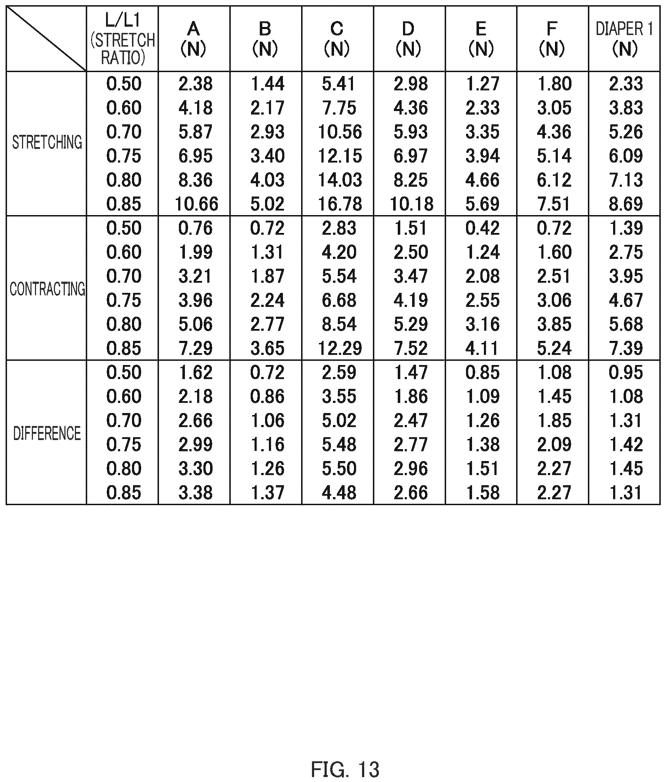

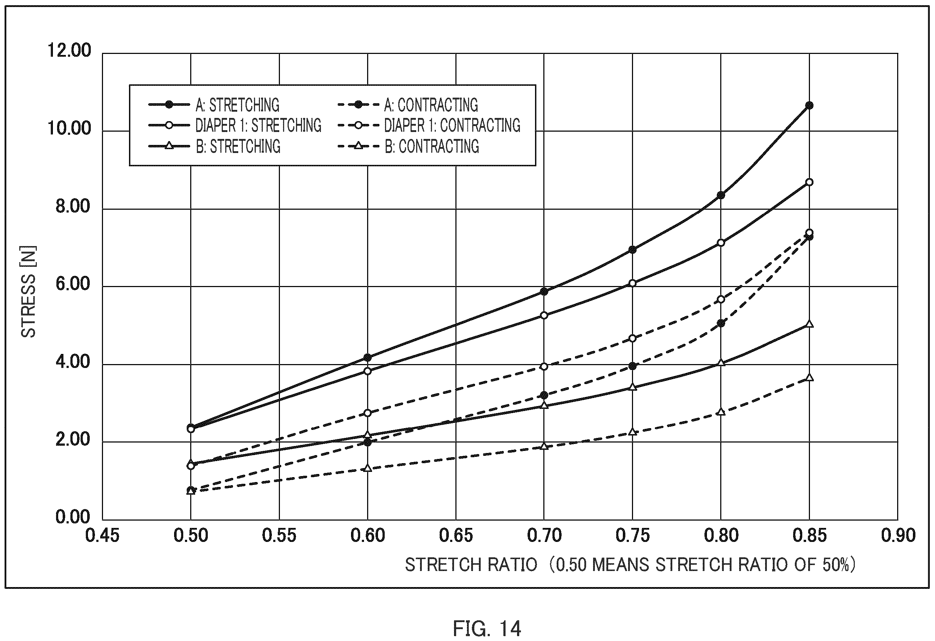

FIG. 11 is a table showing the results of the tensile test on the lower portions FA2 of the diaper 1 and of Comparative Examples A to F. FIG. 12 is a graph showing the relationship between the stretch ratio in the lower portion FA2 of the waist member and the magnitude of force during stretching/contracting for the diaper 1 and Comparative Examples A and B. FIG. 13 is a table showing the results of the tensile test on the upper portions FA1 of the diaper 1 and of Comparative Examples A to F. FIG. 14 is a graph showing the relationship between the stretch ratio in the upper portion FA1 of the waist member and the magnitude of force during stretching/contracting for the diaper 1 and Comparative Examples A and B. In addition, for Comparative Examples A to F, in the same manner as for the diaper 1, the front waist portion (the back waist portion) is divided into the upper portion and the lower portion from the center position in the vertical direction, the tensile test was carried out.

In FIG. 11, the magnitude of force when the lower portion FA2 of the waist member 20 of the diaper 1 is stretched from the natural state to the stretch ratio of 0.80 (80% of the maximum stretched length) is 4.72 N. On the other hand, the magnitude of force when the lower portion FA2 of the waist member 20 is contracted from the maximum stretched state to the stretch ratio of 0.80 is 3.48N. The difference in the magnitude of force between stretching and contraction, 1.24 N (= 4.72 - 3.48), is less than 1.50 N.

On the other hand, in Comparison Example D, the magnitude of force when the waist member is stretched from the natural state to the stretch ratio of 0.80 is 4.33 N, and the magnitude of force when the waist member is contracted from the maximum stretched state to the stretch ratio of 0.80 is 2.79 N. The difference in the magnitude of force between stretching and contraction is 1.54 N (= 4.33 - 2.79). In addition, in all of the comparative examples A to F, the difference in the magnitude of force between stretching and contraction is greater than 1.50 N, which is a larger value than that of the diaper 1.

In other words, in the diaper 1 of the present embodiment, when the waist member 20 is worn in the highly-stretched state (80%), the difference in magnitude of force between stretching and contraction in the lower portion FA2 is 1.5 N or less. The difference in magnitude of force between stretching and contraction is smaller compared to the case of being greater than 1.50 N. Since this lower portion FA2 overlaps with the absorbent main body 10 and the absorbent core 11 (see FIG. 8), the difference in the magnitude of force between stretching and contraction is small in the lower portion FA2. Therefore, it is possible to wear the diaper 1 comfortably while preventing the absorbent main body 10 and the absorbent core 11 from shifting out of position due to the stretching and contraction of the waist member 20 in the lateral direction.

In addition, in FIG. 13, the magnitude of force when the upper portion FA1 of the waist member 20 of the diaper 1 is stretched from the natural state to the stretch ratio of 0.80 is 7.13N. On the other hand, the magnitude of force when the upper portion FA1 of the waist member 20 is contracted from the maximum stretched state to the stretch ratio of 0.80 is 5.68 N. The difference in magnitude of force between stretching and contraction is 1.45 N (= 7.13 - 5.68).

Therefore, when the waist member 20 is worn in the highly-stretched state (80%), the difference in force between stretching and contracting in the lower portion FA2 of the waist member 20 (1.24 N) is smaller than the difference in force between stretching and contracting in the upper portion FA1 of the waist member 20 (1.45 N). This allows the comfortable feeling of the waist member 20 to be maintained. Simultaneously, in the lower portion FA2, which overlaps with the absorbent main body 10 and the like, a large stretching force in the lateral direction is prevented from acting on the absorbent main body 10, making the absorbent main body 10 less likely to shift out of position.

Also, in FIG. 13, the magnitude of force when the upper portion FA1 of the waist member 20 of diaper 1 is stretched from the natural state to the stretch ratio of 0.85 is 8.69 N, and the magnitude of force when the upper portion FA1 of the waist member 20 is contracted from the maximum stretched state to the stretch ratio of 0.85 is 7.39 N. The difference in magnitude of force between stretching and contraction is 1.3 N (= 8.69 - 7.39), which is less than 1.35 N.

On the other hand, in Comparison Example B, the magnitude of force when the waist member is stretched from the natural state until the stretch ratio reaches 0.85 is 5.02 N, and the magnitude of force when the waist member is contracted from the maximum stretched state until the stretch ratio reaches 0.80 is 3.65 N. The difference in magnitude of force between stretching and contraction is 1.37 N (= 5.02 - 3.65). In addition, in all of the comparative examples A to F, the difference in the magnitude of force between stretching and contraction is greater than 1.35 N, which is a larger value than that of the diaper 1.

In other words, even when the diaper 1 is worn in the maximum stretched state (85%) expected in normal use of the waist member 20, the difference in magnitude of force between stretching and contracting is 1.35 N or less in the upper portion FA1 of the waist member 20, where the wearer is likely to feel constriction. compared to the case where the difference in magnitude of force is greater than 1.35 N, the constriction force is weaker, making it easier to wear the diaper 1 comfortably.

Also, in FIG. 11, the magnitude of force when the lower portion FA2 of the waist member 20 of the diaper 1 is stretched from the natural state to the stretch ratio of 0.50 is 1.46 N, and the magnitude of force when it is contracted from the maximum stretched state to the stretch ratio of 0.50 is 0.77 N. When being worn in the low-stretched state (50%), the value obtained by dividing the force during contracting by the force during stretching is 52.7% (= 0.77/1.46), which is greater than 50%.

On the other hand, in Comparison Example A, the magnitude of force when the waist member is stretched from the natural state until the stretch ratio reaches 0.50 is 2.19 N, and the magnitude of force when it is contracted from the maximum stretched state until the stretch ratio reaches 0.50 is 0.94 N. The value obtained by dividing the force during contracting by the force during stretching is 42.9% (= 0.94/2.19). In addition, in all of the Comparative Examples A to F, the value obtained by dividing the force during contracting by the force during stretching is less than 50%, which is smaller than that of the diaper 1.

In other words, in the lower portion FA2 of the waist member 20 of the diaper 1, the magnitude of force acting during contraction is 50% or more of the force acting during stretching. Compared to the case of being less than 50%, the force during contraction is less likely to become excessively small. Therefore, even if the diaper 1 is worn in the low-stretched state (50%), in the lower portion FA2, which is overlap with the absorbent main body 10, it is possible to prevent a shortage of the constriction force exhibited by the waist member 20, which causes the absorbent main body 10 to shift out of position.

Also, in FIG. 11, the magnitude of force when the lower portion FA2 of the waist member 20 is stretched from the natural state to the stretch ratio of 0.60 is 2.39 N, and the magnitude of force when it is contracted from the maximum stretched state to the stretch ratio of 0.50 is 1.56 N. When being worn in the low-stretched state (50%), the value obtained by dividing the force during contracting by the force during stretching is 65.3% (= 1.56/2.39), which is greater than 60%.

On the other hand, in Comparison Example A, the magnitude of force when the waist member is stretched from the natural state until the stretch ratio reaches 0.60 is 3.38 N, and the magnitude of force when it is contracted from the maximum stretched state until the stretch ratio reaches 0.60 is 1.87 N. The value obtained by dividing the force during contracting by the force during stretching is 55.3% (= 1.87/3.38). In addition, in all of the Comparative Examples A to F, the value obtained by dividing the force during contracting by the force during stretching is less than 60%, which is smaller than that of the diaper 1.

In other words, in the lower portion FA2 of the waist member 20 of the diaper 1, the magnitude of force acting during contraction is 60% or more of the force acting during stretching. Compared to the case of being less than 60%, the force during contraction is less likely to become excessively small. Therefore, even if the diaper 1 is worn with the stretch ratio of the waist member 20 at 60%, which is slightly higher than 50%, in the lower portion FA2, which is overlap with the absorbent main body 10, it is possible to prevent a shortage of the constriction force exhibited by the waist member 20, which causes the absorbent main body 10 to shift out of position.

### Others

The above embodiment of the present disclosure is simply to facilitate understanding of the present disclosure and is not in any way to be construed as limiting the present disclosure. The present disclosure may variously be changed or altered without departing from its gist and encompass equivalents thereof.

In the above embodiment, as an example of the underpants-shaped absorbent article, there is described the diaper 1 in which the skin-side sheet 21 of the waist member 20 is configured in an integrated manner continuously from the one side (front side) to the other side (back side) in the longitudinal direction (see FIG. 3 and the like). But the skin-side sheet 21 may be configured as separate members on the front and back sides. For example, a so-called three-piece type underpants-shaped absorbent article may be acceptable which consists of three parts: a front exterior member (front waist portion), a back exterior member (back waist portion), and an absorbent main body.

In the waist member 20 of the diaper 1, a post-handling tape 80 may be provided at a predetermined position. The post-handling tape 80 is a substantially rectangular tape-like member (tape member) elongated in the vertical direction (longitudinal direction), and is made of a resin material such as polyethylene or polypropylene, for example. In the diaper 1, as shown in FIGS. 2 and 3, the post-handling tape 80 is provided on the non-skin-side surface of the back waist portion BA of the waist member 20 (the non-skin-side surface of the non-skin-side sheet 23). The post-handling tape 80 is fixed to the waist member 20 on the one side in the vertical direction and has a sticky portion on the other side in the vertical direction. An adhesive is applied to the sticky portion, and before the diaper 1 is used, a portion of the post-handling tape 80 is in a state of being folded with the sticky portion on the inside, thus preventing exposure of the sticky portion to the outside and protecting a sticky surface.

When discarding of the diaper 1 after use, the diaper 1 is rolled up in the vertical direction such that the absorbent main body 10 faces inward, and the folded post-handling tape 80 is pulled out to expose the sticky portion, and the sticky portion side is wrapped around the diaper 1. Accordingly, the diaper 1 can be held in the rolled state, and the diaper 1 can be discarded without allowing the leakage of excrement and the like adhering to the inside (absorbent main body 10) of the diaper 1.

### REFERENCE SIGNS LIST

1 diaper (underpants-shaped absorbent article),
1a waist opening, 1b leg opening,
10 absorbent main body,
11 absorbent core, 11b core-wrapping sheet,
12 top sheet, 13 back sheet, 15 side sheet, 15t end portion,
20 waist member,
20Ff front fold-back portion, 20Fb back fold-back portion,
21 skin-side sheet, 22 non-skin-side sheet, 23 non-skin-side sheet, 24 cover sheet, 25 waist elastic member,
27 leg elastic member, 27a straight portion, 27b curved portion,
30 leak-proof wall portion,
35 leak-proof-wall elastic member,
40 side joining portion,
50s welded portion, 50s welded portion pair,
60 welded portion row,
70 adhesive portion, 75 adhesive surface,
80 post-handling tape,
91 intermittent portion, 92 intermittent portion,
E1 first elastic member, E2 second elastic member,
CL center position,
FA front waist portion, FA1 front upper portion, FA2 front lower portion, FAC intermediate position,
BA back waist portion,
CA crotch portion,
FL20f front bending position, FL20b back bending position,

## Claims

1. An underpants-shaped absorbent article having a vertical direction, a lateral direction, and a front-back direction that intersect with each other,
the underpants-shaped absorbent article comprising:
a liquid-absorbent absorbent main body; and
a waist member that has stretchability in the lateral direction,
the waist member having
a front waist portion provided on a front side in the front-back direction and
a back waist portion provided on a back side in the front-back direction,
the front waist portion and the back waist portion being joined to each other by a pair of side joining portions that are provided on two sides in the lateral direction,
concerning a magnitude of force when the waist member is stretched in the lateral direction from a natural state until a length of the waist member reaches to 80% of a most stretched length, and
concerning a magnitude of force when the waist member is contracted in the lateral direction from the most stretched state until the length of the waist member reaches 80% of the most stretched length,
a difference between the magnitude of force when being stretched and the magnitude of force when being contracted being 4.0 N or less.

2. The underpants-shaped absorbent article according to claim 1, wherein
concerning a magnitude of force when the waist member is stretched in the lateral direction from the natural state until the length of the waist member reaches to 60% of the most stretched length, and
concerning a magnitude of force when the waist member is contracted in the lateral direction from the most stretched state until the length of the waist member reaches 60% of the most stretched length,
a difference between the magnitude of force when being stretched and the magnitude of force when being contracted is 2.5 N or less.

3. The underpants-shaped absorbent article according to claim 1 or 2, wherein
concerning a magnitude of force when the waist member is stretched in the lateral direction from the natural state until the length of the waist member reaches to 85% of the most stretched length, and
concerning a magnitude of force when the waist member is contracted in the lateral direction from the most stretched state until the length of the waist member reaches 85% of the most stretched length,
a difference between the magnitude of force when being stretched and the magnitude of force when being contracted is 4.0 N or less.

4. The underpants-shaped absorbent article according to claim 3, wherein
concerning a magnitude of force when the waist member is contracted in the lateral direction from the most stretched state until the length of the waist member reaches 50% of the most stretched length, and
concerning a magnitude of force when the waist member is stretched in the lateral direction from the natural state until the length of the waist member reaches to 50% of the most stretched length,
a ratio obtained by dividing the magnitude of force when being contracted by the magnitude of force when being stretched is 50% or more.

5. The underpants-shaped absorbent article according to claim 3, wherein
concerning a magnitude of force when the waist member is contracted in the lateral direction from the most stretched state until the length of the waist member reaches 60% of the most stretched length, and
concerning a magnitude of force when the waist member is stretched in the lateral direction from the natural state until the length of the waist member reaches to 60% of the most stretched length,
a ratio obtained by dividing the magnitude of force when being contracted by the magnitude of force when being stretched is 60% or more.

6. The underpants-shaped absorbent article according to claim 3, wherein
a rate of increase in a magnitude of force when the waist member is stretched in the lateral direction from 50% of the most stretched length to 85% of the most stretched length is 30 N or less.

7. The underpants-shaped absorbent article according to claim 6, wherein
a rate of increase in a magnitude of force when the waist member is stretched in the lateral direction from 75% of the most stretched length to 85% of the most stretched length is 44 N or less.

8. The underpants-shaped absorbent article according to claim 3, wherein
concerning the magnitude of force when the waist member is stretched in the lateral direction from the natural state until the length of the waist member reaches to 80% of the most stretched length, and
concerning the magnitude of force when the waist member is contracted in the lateral direction from the most stretched state until the length of the waist member reaches 80% of the most stretched length,
a difference between the magnitude of force when being stretched and the magnitude of force when being contracted, in a region located below a center position of the pair of side joining portions in the vertical direction, is 1.5 N or less.

9. The underpants-shaped absorbent article according to claim 8, wherein
concerning the magnitude of force when the waist member is stretched in the lateral direction from the natural state until the length of the waist member reaches 80% of the most stretched length, and
concerning the magnitude of force when the waist member is contracted in the lateral direction from the most stretched state until the length of the waist member reaches 80% of the most stretched length,
the difference between the magnitude of force when being stretched and the magnitude of force when being contracted, in the region located below the center position of the pair of side joining portions in the vertical direction
is smaller than
a difference between the magnitude of force when being stretched and the magnitude of force when being contracted, in a region located above the center position of the pair of side joining portions in the vertical direction.

10. The underpants-shaped absorbent article according to claim 3, wherein
in a region located above a center position of the pair of side joining portions in the vertical direction,
concerning a magnitude of force when the waist member is stretched in the lateral direction from the natural state until the length of the waist member reaches to 85% of the most stretched length, and
concerning a magnitude of force when the waist member is contracted in the lateral direction from the most stretched state until the length of the waist member reaches 85% of the most stretched length,
a difference between the magnitude of force when being stretched and the magnitude of force when being contracted is 1.35 N or less.

11. The underpants-shaped absorbent article according to claim 3, wherein
in a region located below a center position of the pair of side joining portions in the vertical direction,
concerning a magnitude of force when the waist member is contracted in the lateral direction from the most stretched state until the length of the waist member reaches 50% of the most stretched length, and
concerning a magnitude of force when the waist member is stretched in the lateral direction from the natural state until the length of the waist member reaches to 50% of the most stretched length,
a ratio obtained by dividing the magnitude of force when being contracted by the magnitude of force when being stretched is 50% or more.

12. The underpants-shaped absorbent article according to claim 3, wherein
in a region located below a center position of the pair of side joining portions in the vertical direction,
concerning a magnitude of force when the waist member is contracted in the lateral direction from the most stretched state until the length of the waist member reaches 60% of the most stretched length, and
concerning a magnitude of force when the waist member is stretched in the lateral direction from the natural state until the length of the waist member reaches to 60% of the most stretched length,
a ratio obtained by dividing the magnitude of force when being contracted by the magnitude of force when being stretched is 60% or more.

13. The underpants-shaped absorbent article according to claim 3, wherein
the waist member has a plurality of elastic members between a first sheet and a second sheet that are joined by a plurality of welded portions,
the plurality of elastic members being arranged spaced apart in the vertical direction,
the plurality of elastic members being capable of stretching and contracting in the lateral direction, and
each of the elastic members is attached to the waist member, in a state of being contracted in the lateral direction, by being sandwiched between two of the plurality of welded portions that are adjacent in the vertical direction.
